# EUROPEAN PATENT SPECIFICATION

(11) **EP 3 166 651 B1**
(45) Date of publication and mention of the grant of the patent: **05.06.2019**
(21) Application number: 15818835.9
(22) Date of filing: 07.07.2015
(51) Int. Cl.: A61L 27/54, A61L 27/02, A61L 27/12, A61L 24/02, A61L 24/00

(54) **INJECTABLE BONE SUBSTITUTES FOR AUGMENTING IMPLANT FIXATION**
INJIZIERBARE KNOCHENERSATZMATERIALIEN ZUR VERSTÄRKUNG DER IMPLANTATFIXIERUNG
SUBSTITUTS OSSEUX INJECTABLES POUR AUGMENTER LA FIXATION DE PROTHÈSES

(30) Priority: 10.07.2014 EP 14176540
(43) Date of publication of application: 17.05.2017
(73) Proprietor: Bone Support AB, 223 70 Lund (SE)
(72) Inventor: KASIOPTAS, Argyrios, S-22651 Lund (SE); LIDÉN, Eva Christina, S-22471 Lund (SE); LINDBERG, Björn Fredrik, S-22363 Lund (SE)
(74) Representative: Guardian IP Consulting I/S
(86) International application number: PCT/SE2015/050807
(87) International publication number: WO 2016/007080

(56) References cited:
- EP-A1- 2 353 619
- WO-A1-2004/078223
- WO-A1-2004/078223
- WO-A1-2011/098438
- WO-A1-2014/128217
- SAKAMOTO YOSHIAKI ET AL: "Mechanical Strength and In Vitro Antibiotic Release Profile of Antibiotic-Loaded Calcium Phosphate Bone Cement", THE JOURNAL OF CRANIOFACIAL SURGERY, LIPPINCOTT WILLIAMS & WILKINS, US, vol. 24, no. 4, 1 July 2013 (2013-07-01), pages 1447-1450, XP008183498, ISSN: 1049-2275, DOI: 10.1097/SCS.0B013E31829972DE
- ONDER KILICOGLU ET AL: "Effect of antibiotic loading on the shear strength at the stem-cement interface (Shear strength of antibiotic loaded cement)", INTERNATIONAL ORTHOPAEDICS, SPRINGER, BERLIN, DE, vol. 32, no. 4, 14 March 2007 (2007-03-14) , pages 437-441, XP019624461, ISSN: 1432-5195
- MARTHA GEFFERS ET AL: "Reinforcement Strategies for Load-Bearing Calcium Phosphate Biocements", MATERIALS, vol. 8, no. 5, 20 May 2015 (2015-05-20), pages 2700-2717, XP055422600, DOI: 10.3390/ma8052700
- KILICOGLU O ET AL.: 'Effect of antibiotic loading on the shear strength at the stem-cement interface (Shear strength of antibiotic loaded cement' INTERNATIONAL ORTHOPAEDICS (SICOT vol. 32, 2008, pages 437 - 441, XP019624461
- EHRENBORG C ET AL. MECHANICAL PROPERTIES OF A CERAMIC BONE SUBSTITUTE LOADED WITH GENTAMICIN 10 May 2012, XP055349853
- YANG XU ET AL.: 'In Vitro Elution Characteristics of Vancomycin in a Composite Calcium Phosphate/Calcium Sulfate Bone Substitute' HSSJ vol. 8, 2012, pages 129 - 132, XP035091113
- SAKAMOTO Y ET AL.: 'Mechanical Strength and In Vitro Antibiotic Release Profile of Antibiotic-Loaded Calcium Phosphate Bone Cement' THE JOURNAL OF CRANIOFACIAL SURGERY vol. 24, no. 4, 2013, pages 1447 - 1450, XP008183498
- YANG YU-HUI ET AL.: 'Adding vancomycin to bone cement: research on its influence on mechanical fixation strength using rabbit femoral prostheses' ORTHOPAEDIC SURGERY vol. 3, no. 4, 2011, pages 265 - 267, XP055336573
- WANG JIAN-SHENG ET AL.: 'Factors affecting the static shear strength of the prosthetic stem-bone cement interface' JOURNAL OF MATERIALS SCIENCE : MATERIALS IN MEDICINE vol. 14, 2003, pages 55 - 61, XP055336576

## Description

### Technical field of the invention

The present invention relates to the use of a cyclic glycopeptide to enhance the resistance of a bone substitute composition to one or more of a tensile, shear and torsional force. The bone substitute composition, on mixing forms an injectable and/or moldable, and hardenable composition for use in orthopedic surgery, where the composition is employed in combination with an implant to enhance the fixation of the implant, thereby enhancing bone re-growth required for stabilization of the implant.

### Background of the invention

Until the last century, physicians relied on casts and splints to support and stabilize a bone from outside the body. The advent of sterile surgical procedures has reduced the risk of infection, allowing doctors to internally set and stabilize fractured bones. Implants are now widely used in orthopedic surgery, for the repair of broken bones, as well as in joint arthroplasty. Internal fixation serves to stabilize and support a broken bone until it is strong enough to support the body's weight and movement. Internal fixation allows shorter hospital stays, enables patients to return to function earlier, and reduces the incidence of nonunion (improper healing) and malunion (healing in improper position) of broken bones.

During a surgical procedure to set a fracture, the bone fragments are first repositioned (reduced) into their normal alignment. They are then held in place with special implants, such as plates, screws, pins, nails and wires etc. Screws are used for internal fixation more frequently than any other type of implant. Although the screw is a simple device, there are different designs based on the type of fracture and how the screw will be used. Screws come in different sizes for use with bones of different sizes. Screws can be used alone to hold a fracture, as well as with plates, rods, or nails. Plates serve as internal splints that are attached to the bone with screws and serve to hold the broken pieces of bone together. After the bone heals, screws may be either left in place or removed.

In some fractures of the long bones (e.g. femur and tibia), the bone pieces can be held together by inserting a rod or nail through the hollow center of the bone. Screws at each end of the rod are used to keep the fracture from shortening or rotating, and also for holding the rod in place until the fracture has healed. Rods and screws may be left in the bone after healing is complete.

The implants used for internal fixation are commonly made from stainless steel and titanium, which are durable and strong, however their inherent strength cannot contribute to achieving a strong initial fixation if inserted into partly weak bone. The use of screws to position and stabilize fractured bones in the correct alignment requires a strong initial fixation to reduce the risk of premature device detachment and delayed fracture healing. This is particularly the case where screws are used to position plates or rods, where postoperatively the screw will be subjected to pull-out forces, which may be composed of tension and/or shear stresses that may lead to screw loosening or complete detachment. Some weeks following surgery, bone growth and fracture healing lead to formation of bone with sufficient mechanical strength, such that the inserted screws and plates can be removed, if deemed necessary.

Polymethylmethacrylate (PMMA) has been used to reinforce the fixation of screws, in particular pedicle screws used in vertebral reconstruction surgery of patients suffering from osteoporosis. A number of disadvantages are associated with using synthetic polymers such as PMMA in bone re-construction, particularly in patients suffering from osteoporosis. Firstly, the heat generated during the polymerization of PMMA kills adjacent bone tissue resulting in a soft fibrous interface between an implant and adjacent bone tissue, which leads to poor implant fixation. Secondly, PMMA is rigid and non-compressible, and when used in augmentation of screws inserted into a vertebra of an osteoporotic patient, it greatly increases the risk of fracture in the adjacent vertebra (fulcrum). Additionally, PMMA-type synthetic polymers are not biodegradable and as a result they do not allow for subsequent replacement by bone tissue adjacent to implants. Since inserted screws may subsequently need to be removed, it is important to avoid the use of cements that are nondegradable which may lead to greater bone damage if the screws need to be surgically removed.

Implants are also increasingly used in the treatment of patients needing primary joint prosthetic surgery, as well as replacement prostheses by revision arthroplasty. Revision arthroplasty presents a substantial challenge for the surgeon if the primary prosthesis has been cemented with polymethyl methacrylate (PMMA). PMMA cement is able to interfoliate with cancellous bone, so its removal entails removing large amounts of endogenous bone together with the cured PMMA. If the prosthesis becomes infected, all residual PMMA needs to be removed because of its potential to host bacteria growth. Thus, when performing revision surgery, removal of a PMMA cemented prosthesis can create additional voids and defects in the bone. As a result, it has become increasingly common to perform primary prosthesis operations with cementless prostheses. However, the use of cementless prostheses is not in itself without problems. The most common cause of cementless prosthetic implant failure is aseptic loosening and periprosthetic osteolysis. The impact of immediate aseptic loosening of primary total knee prostheses within the first year following implantation on prosthesis survival rates has been studied by Pijls et al., 2012. The study reveals that for every mm of prosthesis migration (measured as 3D migration on any point on the prosthesis surface using radiostereometric analysis), the need for revision surgery at 5 years increases by 8%.

During the past decade, revision arthoplasty of hips has also increasingly made use of prostheses without cement fixation. The survival rates of cementless revision prostheses are difficult to predict and depend on many factors including the optimum choice of implant size, the anatomy of the femur, and the degree of bone destruction. The accurate assessment of these clinical parameters requires a high degree of surgical experience that is not always available.

From the review article, S. Yoshiaki et al, The Journal of Cranofacial Surgery , vol. 24, n°4, 1 July 2013, p.1447-1450, it can be concluded that, at the priority date, common approaches to improve the mechanical performance of calcium phosphate cements (CPC) include the modification of the cement liquid with polymeric additives, the addition of fibres or the use of dual-setting cements.

In view of the deficiencies in the immediate strong stabilization of implants used in treatment of musculoskeletal disorders, such as a non-cemented prosthesis used in primary and revision arthoplasty, as well as implants used in the fixation of bone fractures, there exists a need for new cements that when used in combination with implants can enhance their fixation immediately following surgery. Improved fixation of implants would benefit a mammal having a musculoskeletal disorder, for example a human, dog, horse or cat.

### Summary of the invention

The invention is directed to the use of a cyclic glycopeptide to enhance the resistance of a composition to one or more of a tensile, shear and torsional force, wherein the composition comprises (or consists essentially of) a powder component and an aqueous liquid component and a cyclic glycopeptide component; wherein the powder component comprises (or consists essentially of) a calcium sulfate component and/or a calcium phosphate component; and wherein the powder component and the aqueous liquid component and the cyclic glycopeptide component on mixing forms an injectable or moldable composition capable of hardening *in vivo.*

In an embodiment of the use of a cyclic glycopeptide according to the invention, the powder component comprises a calcium sulfate component and a calcium phosphate component, which on mixing with the aqueous liquid component and the cyclic glycopeptide component forms an injectable and/or moldable composition.

In one embodiment of said use, the cyclic glycopeptide is selected from the group vancomycin, eremomycin, ristocetin A, teicoplanin, telavancin, bleomycin, ramoplanin, and decaplanin; or more preferably is selected from the group vancomycin, eremomycin, ristocetin A, teicoplanin and televancin.

The ceramic bone substitute composition according to the above use of a cyclic glycopeptide corresponds to the composition for fixation of an implant in a mammal, in respect of each of the features defined above for the composition. Also disclosed is a method for fixation of an implant comprising (or consists essentially of):
a) mixing a dry powder composition consisting essentially of hydroxyapatite in an amount ranging from 30 to 50 wt/wt%, calcium sulfate in an amount ranging from 50 to 70 wt/wt%, and cyclic glycopeptide with an aqueous liquid to form an injectable and/or moldable composition; wherein the amount of cyclic glycopeptide ranges from 1-600mg/ml injectable and/or moldable composition,
b) inserting the resulting composition from step a) into a bone cavity;
c) introducing an implant into the bone cavity either before or after inserting the composition in step b); and
d) allowing the injectable composition to harden *in vivo,* said method is not part of the invention.

In the method for fixation of an implant, the injectable and/or moldable composition comprises between 0.1 and 2 ml, preferably between 0.2 and 0.7 ml of the aqueous liquid per gram bone substitute powder.

In the method for fixation of an implant, the calcium sulfate component comprises α-calcium sulfate hemihydrate and additionally an accelerator selected from among calcium sulfate dihydrate and sodium chloride.

In the method for fixation of an implant, the aqueous liquid may comprise an X- ray contrast agent; and/or one or more therapeutic agent.

### Brief description of the figures

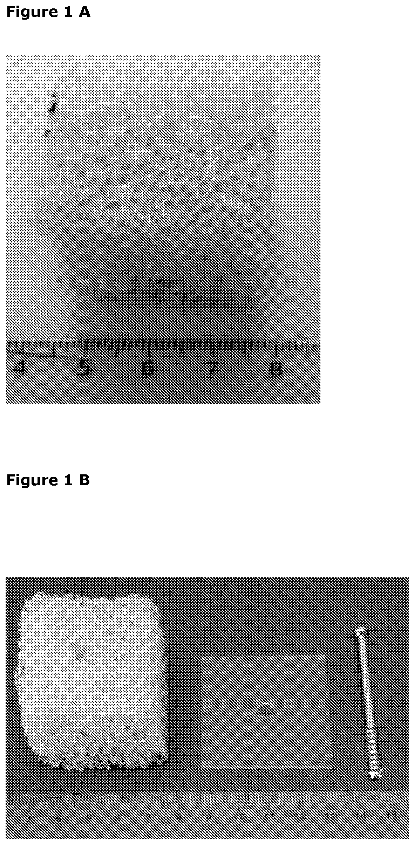

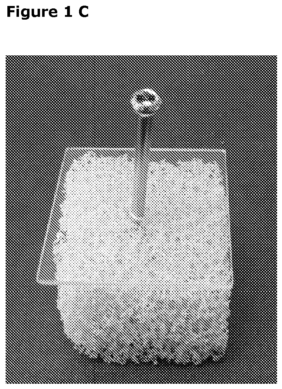

**Figure 1** [A] shows a 81cm³ (4.5x4.5x4 cm³) block cut from the foam block (1522-507 block: open cell 7.5#, 0.12g/cc supplied by Sawbones®); that has an open cell structure and cell size of 1.5 to 2.5 mm resembling that of human cancellous bone. The foam block material has a density of 0.12g/cc, a compressive strength is 0.28 MPa and compressive Modulus is 18.6 MPa. [B] shows the foam block and a 2mm thick plexiglas plate used to simulate the cortical bone, that is perforated by a single hole drilled centrally through the plate, and a cannulated, partially threaded, 5.0x60mm long screw made of steel (Asnis™ III) supplied by Stryker (Footandanklefixation.com). In this stage, a 2cm-deep hole has been drilled in the Sawbones® block with a 3.5mm drill, [C] shows a screw inserted through the Plexiglas plate into the 81cm³ block, through the pre-drilled hole.

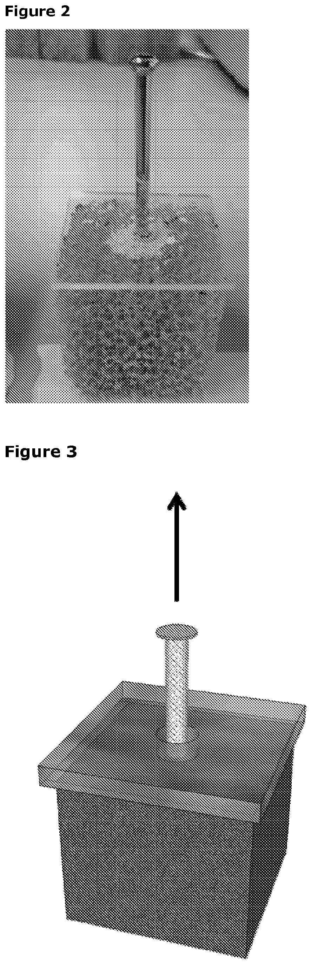

**Figure 2** shows a screw inserted through the Plexiglas plate into the 81cm³ block, where the screw is augmented with a ceramic bone substitute composition. The Plexiglas plate simulates the cortical bone layer that surrounds cancellous bone. The hole in the Plexiglas plate is large enough to allow the screw to pass through without the screw thread engaging with the Plexiglas.
**Figure 3** Cartoon illustrating the axial force exerted on the inserted screw in the pull-out test.

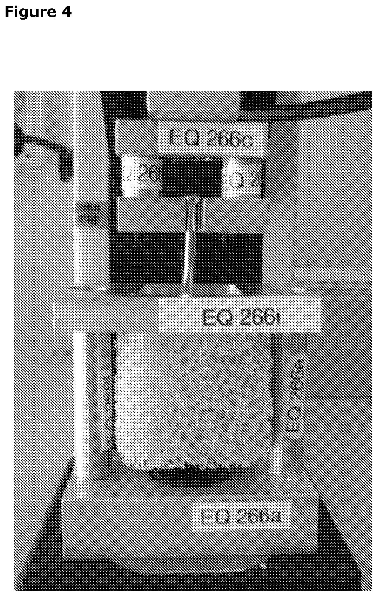

**Figure 4** shows the foam block and inserted screw mounted on the MTS Insight 5 single column material testing workstation, used for measuring the force needed to raise the inserted screw from the block.

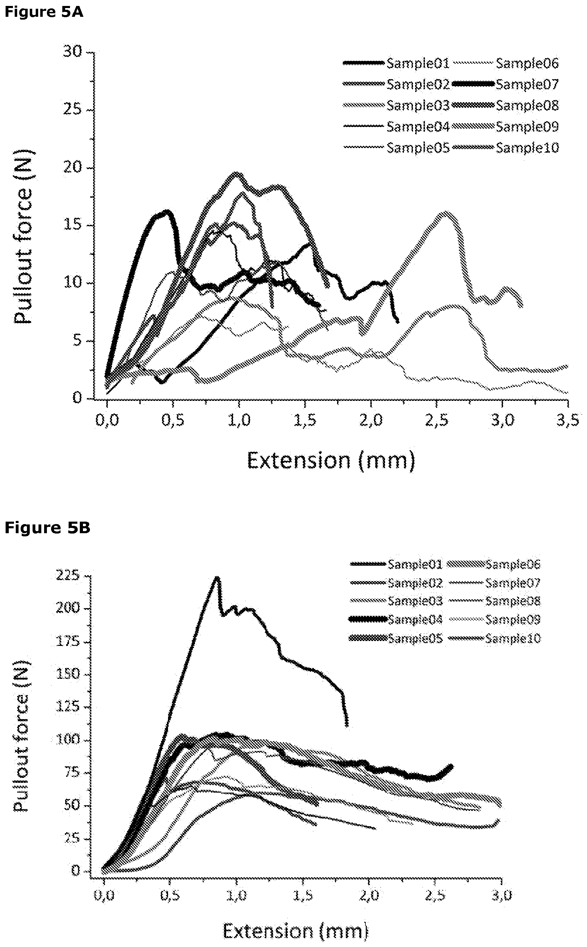

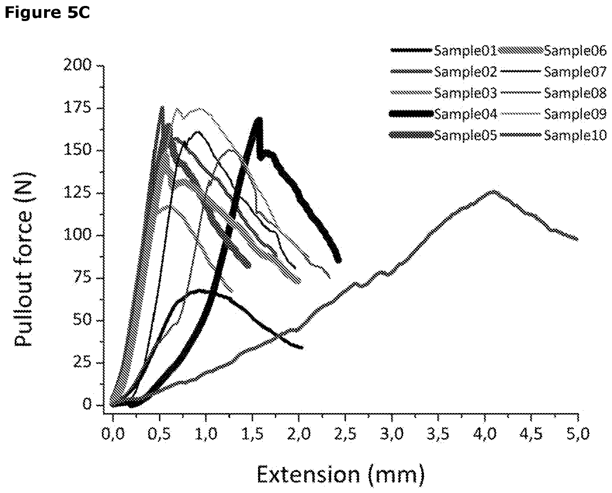

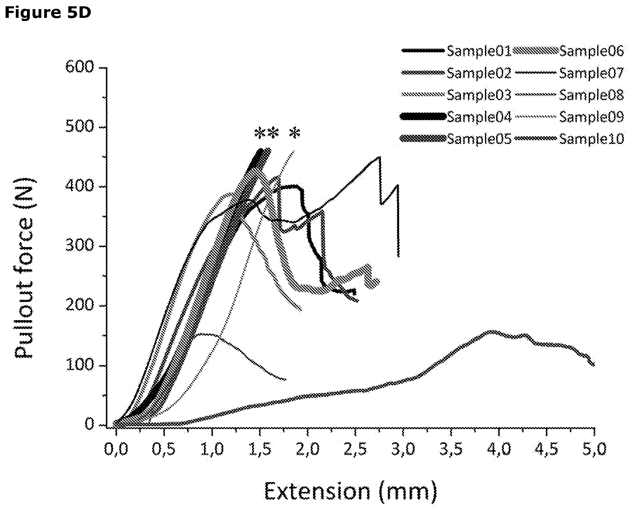

**Figure 5** Profiles of the pull-out force (Newtons) required to raise each of 10 screws inserted in a model foam block that is maintained under wet conditions; where the inserted screw is un-augmented (reference sample) in (A); or augmented with ceramic bone substitute composition of calcium sulfate and hydroxyapatite ("CSH/HA") in (B); or ceramic bone substitute composition supplemented with gentamicin ("CSH/HA + Genta") in (C); or ceramic bone substitute composition supplemented with vancomycin ("CSH/HA + Vanco") in (D). The profiles labeled with * reached the maximum limit of the MTS load cell (500N).

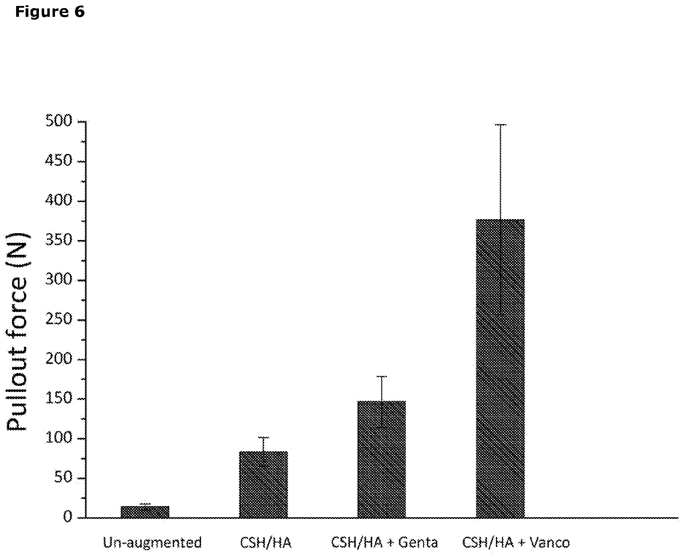

**Figure 6** shows the tensile force (Newtons) required to raise screws inserted in a model foam block that is maintained under wet conditions; where the inserted screw is augmented with either ceramic bone substitute composition ("CSH/HA"); ceramic bone substitute composition supplemented with gentamicin ("CSH/HA + Genta"); or ceramic bone substitute composition supplemented with vancomycin ("CSH/HA + Vanco"). Each bar in the plot represents the mean peak force (with standard deviation) for the 10 samples tested under the specified conditions.

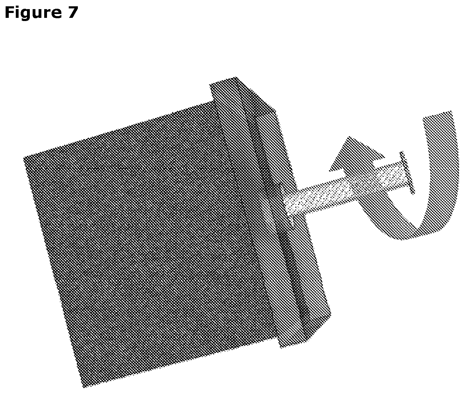

**Figure 7** Cartoon illustrating a method for inducing and measuring torsion force or shear forces on an inserted screw. Torsion forces can be measured by means of a torque driver by unscrewing the implant from the augmented foam.

### Definition of terms:

Augmented: used herein in respect of an implant, refers to an implant that is implanted with a hardenable bone substitute cement to improve fixation in the bone tissue.

Consisting essentially of: this term in respect of each of the method, the composition; the powder component, and the injectable and/or moldable composition of the invention necessarily includes the listed steps and/or ingredients therein, and where each is further open to unlisted steps and/or ingredients that do not materially affect the basic and novel properties of the invention.

Cyclic glycopeptide: is a non-ribosomal cyclic glycopeptide characterized by a large amphotheric organic structure having limited conformational flexibility; water-solubility and the ability to react with acids and bases; as exemplified by cyclic glycopeptide antibiotics, including vancomycin, eremomycin, ristocetin A, teicoplanin, telavancin, bleomycin, ramoplanin, and decaplanin.

Pull-out strength: is the fixation strength of an implant following implantation within bone tissue in a subject; and the corresponding resistance of the implant to detachment from its site of implantation as a result of the various stress forces exerted on the implant during and following implantation. These stress forces may be tensile stress and/or shear stress, each of which can be measured as described herein.

### Detailed description of the invention

Also disclosed but not part of the invention is a composition for use in the treatment of a muscular-skeletal disorder in a mammal receiving an implant to enhance bone re-growth for stabilization of the implant. In contrast to the PMMA cements, the composition is composed of a biphasic ceramic bone substitute, comprising a calcium sulfate component and/or a calcium phosphate component, which are resorbed and promote the in-growth of new bone (Zampelis et al.). The components of the composition, following mixing, form a composition, or paste, that can be injected or molded into the bone cavity into which the implant is to be inserted. Use of the composition of the invention in said treatment provides an unexpectedly strong initial fixation of an inserted implant, such as a screw, pin, nail, rod, wire, plate or stem of an artificial joint, which insures that the precise alignment of fractured bones or replacement joint is maintained during and post-surgery, and that subsequent micro-motion is minimized. Micro-motion of an implant is known to disturb/disrupt bone re-growth following implant surgery, which is important for the long term implant stability in the mammal, and may ultimately lead to implant detachment if not minimized. Accordingly, the composition having the combined properties of promoting bone re-growth and preventing the disruption of this re-growth by micro-motion, leads to an unexpected enhancement of implant fixation and implant stabilization, which is essential for long term treatment of a muscular-skeletal disorder in a mammal receiving an implant.

The composition is intended for the fixation of an implant in a mammal suffering from a musculoskeletal disorder, such as for example a bone fracture or a prosthesis in a mammal in need of joint arthroplasty, where the composition comprises (or consists essentially of) a powder component, a cyclic glycopeptide component and an aqueous liquid component, where the powder component, the cyclic glycopeptide component and the aqueous liquid component on mixing forms a composition capable of *in vivo* hardening. The powder component comprises (or consists essentially of) a calcium sulfate component and/or a calcium phosphate component, which is particularly suitable for use as a ceramic bone substitute composition. The cyclic glycopeptide component can be a dry component, or the liquid component may comprise the cyclic glycopeptide.

The calcium sulfate component may comprise calcium sulfate hemihydrate and be combined with an accelerator, where the accelerator may, for example, be selected from calcium sulfate dihydrate and a suitable salt, such as sodium chloride for *in vivo* hardening of the calcium sulfate hemihydrate by hydration. When the accelerator is sodium chloride, this may suitably be provided in the aqueous liquid, as an aqueous saline solution.

The calcium sulfate hemihydrate may be α- or β-calcium sulfate hemihydrate, where α-calcium sulfate hemihydrate is preferred, and suitably the powdered calcium sulfate hemihydrate has a particle size of less than 500 µm, for example less than 100 µm, or when 99% of the particles have a particle size less than 80 µm.

When calcium sulfate hemihydrate (CSH) is mixed with the aqueous liquid, it will hydrate to calcium sulfate dihydrate (CSD), according to the below reaction scheme (1):

CaSO₄•0.5 H₂O + 1.5 H₂O => CaSO₄ • 2 H₂O + Heat (1)

The accelerator in the bone substitute powder serves to increase the rate of hydration of CSH and its re-crystallization to CSD. When the accelerator is powdered CSD, it has a suitable particle size that is less than 500 µm, for example less than 150 µm, or for example less than 100 µm.

The particulate calcium sulfate dihydrate should be present in an amount between 0.1 and 10 wt/wt%, for example between 0.1 and 2 wt/wt% of the total weight of the bone substitute powder.

The powdered calcium phosphate component may e.g. be amorphous calcium phosphate (ACP), monocalcium phosphate monohydrate (MCPM; Ca(H₂PO₄)•2H₂O), dicalcium phosphate dihydrate DCPD (brushite; CaHPO₄•2H₂O), octacalcium phosphate (Ca₈(HPO₄)₂(PO₄)₄•5H₂O), calcium deficient hydroxyapatite (CDHA; Ca₉(HPO₄)(PO₄)₅(OH)), tricalcium phosphate (TCP; Ca₃(PO₄)₂), and hydroxyapatite (HA; Ca₁₀(PO₄)₆(OH)₂.
It is preferred that the powdered calcium phosphate component is hydroxyapatite or tricalcium phosphate, wherein the hydroxyapatite or α-tricalcium phosphate has a particle size of less than 100 µm. Preferably the HA powder is sintered and micronized and contains more than 90% such as 95% or more, e.g. 99% crystalline HA. The HA powder may have been additionally heat treated at 100-900°C for 10min - 10 h (e.g. at 500°C for 2 h), as described in (PCT/EP2014/053330).

When the powdered calcium phosphate component is tricalcium phosphate, it is advantageous to add an accelerator, known *per se,* such as hardened particulate calcium phosphate. The hardened particulate calcium phosphate should have a particle size which is less than 100 µm, suitably less than 50 µm, and comprise between 0.1 and 10 wt/wt%, for example between 0.5 and 5 wt/wt% of the calcium phosphate in the bone substitute powder.

The reaction of calcium phosphate to hardened calcium phosphate can also be accelerated by addition of a phosphate salt, for example disodium hydrogen phosphate (Na₂HPO₄), which may be added as dry particles or dissolved in the aqueous liquid. In this case, the accelerator should be present in the aqueous liquid at concentrations of 0.1-10 wt%, for example 1-5 wt%.

In order to confer an initial strength to the hardened ceramic bone substitute composition, the calcium sulphate hemihydrate may comprise 2-80 wt%, preferably 10-30 wt% of the dry powder to be mixed with an aqueous liquid, when a calcium phosphate to be hardened is used. Likewise, when the calcium phosphate is to be converted to hardened calcium phosphate it should comprise 20-98 wt%, preferably 70-90 wt% of the dry powder. When using hydroxyapatite as the calcium phosphate component, the hydroxyapatite suitably comprises from 30 to 50 wt% of the dry powder, such as about 40 wt%, in which case the CSH +CSD will constitute from 50 to 70 wt% of the dry powder, such as about 60 wt%.

The composition further comprises a non-ribosomal cyclic glycopeptide, where the cyclic glycopeptide may be included in the aqueous liquid component or may be provided as a dry component of the composition. The addition of cyclic glycopeptide to the composition has been found to produce an injectable composition that when introduced into a bone cavity with an implant can significantly increase the fixation strength of the inserted implant. The enhanced fixation measured for implants augmented with the composition comprising cyclic glycopeptide is observed immediately following *in vivo* hardening of the composition, where hardening occurs within 20 minutes from start of mixing the cyclic glycopeptide-containing composition. The fixation strength of augmented implants provided by use of the composition of the invention can be determined by measuring the pull-out strength of the implant. Pull-out strength comprises a combination of increased axial tensile strength and shear strength. *In vitro* methods for measuring the pull-out strength and resistance to torsional forces of implants augmented with the composition of the invention are described in example 2.

Non-ribosomal cyclic glycopeptide of the invention include vancomycin, eremomycin ristocetin A, teicoplanin, telavancin, bleomycin, ramoplanin and decaplanin or a combination thereof; preferably any one of eremomycin, ristocetin A, teicoplanin, and telavancin or a combination thereof. In a preferred embodiment the cyclic glycopeptide is vancomycin, which in common with other cyclic glycopeptides are water soluble and able to react with acids and bases and they are characterized by their large amphotheric organic structure having limited conformational flexibility (Liskamp et al 2008). Vancomycin in the composition is preferably provided in the form of vancomycin hydrochloride. In view of the properties of these cyclic glycopeptides, such as vancomycin, it is theorized that one or more functional group(s) of these cyclic glycopeptides interacts with components of the composition, such as to increase its structural strength when hardened, both in respect of its tensile, shear and torsional strength. Importantly, interactions between cyclic glycopeptides (e.g. vancomycin) and the other components of the composition that increase its tensile, shear, and torsional strength on hardening, take place when the cement is maintained in wet conditions and at temperatures (circa 35-42°C, e.g. 37°C), corresponding to conditions within a bone cavity in vivo into which the composition is inserted. Interaction (e.g. ionic and/or chemical bonding) occurring between functional group(s) of these cyclic glycopeptides with bone tissue functional groups in the bone cavity will contribute to the enhanced fixation of an implant when augmented with a composition of the invention.

The composition comprising a ceramic bone substitute combined with cyclic glycopeptide has particularly advantages when used in the treatment of musculoskeletal disorders in mammals additionally suffering from osteopenia or osteoporosis. Injectable and/or moldable compositions (pastes) of the invention that are injected or molded into an osteoporotic mammal are less likely to damage adjacent bone tissue since the material strength and stiffness more closely matches that of the mammal's own bone tissue in contrast to the acrylate-based cements such as PMMA. The composition of the invention is useful for the treatment of a human, dog, cat or horse having a musculoskeletal disorder; and in particular the treatment of a human.

A suitable aqueous solution comprising cyclic glycopeptide (e.g. vancomycin) is one comprising 2 - 1250mg vancomycin hydrochloride/ml solution, for example at least 5, 10, 20, 40, 60, 80 100, 120, 140, 160, 180, 200, 300, 400, 600, 800, 1000 mg cyclic glycopeptide (e.g. vancomycin hydrochloride)/ml solution. For example, a suitable aqueous solution comprising cyclic glycopeptide (e.g. vancomycin) is one comprising 30 - 50; 50 - 70, 70 - 90, 90 - 120, 120 - 130, 130 - 150, 150 - 170, 170 - 190, 190 - 210, 210 - 250, 250-500, 500-750, 750-1000mg cyclic glycopeptide (e.g. vancomycin hydrochloride)/ml solution.

A composition comprising a bone substitute powder component and an aqueous liquid component that are mixed together to form an injectable composition (paste), having a cyclic glycopeptide (e.g. vancomycin) content of 1 - 600mg cyclic glycopeptide (e.g. vancomycin)/ml paste, for example at least, or no more than: 5, 10, 15, 20, 40, 60, 80 100, 120, 140, 160, 180, 250, 275, 300, 325, 350, 375, 400, 425, 450, 500, 525, 550, 575 and 600mg cyclic glycopeptide (e.g. vancomycin)/ml paste. For example, a suitable paste is one comprising 5 - 15; 15 - 25; 25 - 35; 35 - 45; 45-55, 55 - 65, 65 - 75, 75 - 85, 85 - 105, 105 - 125, 125 - 145, 145 - 165, 165 - 185, 185 - 205, 205 - 215, 215 - 225, 225-250, 250-300, 330-350, 350-400, 450-500, 500-550, 550-600mg cyclic glycopeptide (e.g. vancomycin)/ml paste. When the injectable bone substitute composition (paste) is prepared from a powder comprising (or consisting essentially of) 50 to 70% wt/wt% (for example 60 wt/wt%) calcium sulfate component (CSH +CSD) and 30 to 50 wt/wt% (for example 40 wt/wt%) hydroxyapatite, and a liquid component, then the paste comprises 5 - 15; 15 - 25; 25 - 35; 35 - 45; 45-55, 55 - 65, 65 - 75, 75 - 85, 85 - 105, 105 - 125, 125 - 145, 145 - 165, 165 - 185, 185 - 205, 205 - 215, 215 - 225, 225-250, 250-300, 330-350, 350-400, 450-500, 500-550, 550-600mg cyclic glycopeptide (e.g. vancomycin)/ml paste. Preferably the paste has a cyclic glycopeptide (e.g. vancomycin) content of from 20 - 150, 20 - 140, 20 - 120, 20 - 100, 20 - 80, 20 - 60, 30 - 150, 30 - 140, 30 - 130, 30 - 120, 30 - 110, 30 - 100, 30 - 80, 40 - 150, 40 - 140, 40 - 130, 40 - 120, 40 - 110, 40 - 100mg cyclic glycopeptide (e.g. vancomycin)/ml paste. Pastes having this composition all showed acceptable setting performance for an injectable bone substitute; compatible with surgical procedures for the treatment of muscular skeletal disorders employing the fixation of implants using the paste (Example 3).

The aqueous liquid component may further include an X-ray contrast agent, such as agents described in US8,586,101 and US5,447,711, including iotrolan, ioxaglate, iodecimol, and iosarcol. Suitably, the agent is a non-ionic, low-osmolarity, water-soluble contrast agent, for example an iodine-containing aqueous liquid, such as iohexol, iodixanol, ioversol, iopamidol, and iotrolane. As an alternative to water soluble non-ionic X-ray contrast agents, biodegradable particles comprising biocompatible and biodegradable X-ray contrast agent, as disclosed in WO 2009/081169, may be used to provide radiopacity in the bone substitute of the present invention. The aqueous liquid may include sodium chloride, such as 0.9 w/v% sodium chloride, to act as an accelerant.

The mixing ratio for the powder and the aqueous liquid component is called the liquid-to-powder ratio (L/P). The aqueous liquid in the ceramic bone substitute composition should comprise between 0.1 and 2 mL/g powder, for example between 0.2 and 0.7 mL/g or between 0.3 and 0.5 mL/g. A lower L/P ratio, such as between 0.2 and 0.4 mL/g can be employed to reduce the setting time, however a lower L/P ratio may compromise the injectability of the composition.

In one embodiment, the powder consists of 60 wt/wt% of a calcium sulfate component and 40 wt/wt% hydroxyapatite, where the calcium sulfate component consists of 59.6 wt/wt% calcium sulfate hemihydrate and 0.4 wt/wt% calcium sulfate dihydrate, and the liquid component comprises 100 - 250mg cyclic glycopeptide (e.g. vancomycin)/ml solution.

Additives to be included in the composition, either by addition to the bone substitute powder or the aqueous liquid include one or more therapeutic agents, such as antimicrobial drugs, chemotherapeutics, vitamins, hormones, cytostatics, bisphosphonates, growth factors, proteins, peptides, bone marrow aspirate, platelet rich plasma and demineralised bone. Antibiotics suitable for inclusion in the composition are one or more of belonging to the group consisting of glycoside antibiotics, the group consisting of penicillins, the group consisting of cephalosporins, the group consisting of antifungal drugs, or the antibiotic agent is rifampicin or clindamycin. Preferably, the antibiotic agent(s) is/are selected from the list consisting of: gentamicin, tobramycin, cefazolin, rifampicin, clindamycin, nystatin, griseofulvin, amphotericin B, ketoconazole and miconazole. Additional additives (composition components) suitable for inclusion in the composition include one or more viscosity modifying agent.

The invention relates to the use of a non-ribosomal cyclic glycopeptide to enhance the resistance of a composition to a stress force such as a tensile, shear and torsional force, wherein the composition comprises a powder component and an aqueous liquid component, further including a cyclic glycopeptide component; wherein the powder component comprises a calcium sulfate component and a calcium phosphate component; and wherein the powder component and the aqueous liquid component and the cyclic glycopeptide component on mixing form an injectable composition capable of *in vivo* hardening. The cyclic glycopeptide component can be provided as a dry component, or can be provided as a component of the aqueous liquid component. The cyclic glycopeptide component may be selected from among vancomycin, eremomycin, ristocetin A, teicoplanin, telavancin, bleomycin, ramoplanin, and decaplanin; more preferably selected from among vancomycin, eremomycin, ristocetin A, teicoplanin, and telavancin. Vancomycin is preferably provided in the form of vancomycin hydrochloride. The calcium sulfate component may comprise α-calcium sulfate hemihydrate and additionally an accelerator, wherein the accelerator is selected from among calcium sulfate dihydrate and sodium chloride. The calcium phosphate component may be hydroxyapatite or alternatively tricalcium phosphate and a phosphate salt or a hardened calcium phosphate. The bone substitute powder may consist essentially of from 50 to 70% wt/wt% (for example 60 wt/wt%) calcium sulfate component (CSH +CSD) and 30 to 50 wt/wt% (for example 40 wt/wt%) hydroxyapatite, and the liquid component may comprise 2-1250 mg vancomycin hydrochloride/ml solution. The ceramic bone substitute composition comprises between 0.1 - 2 , 0.1 - 1.9, 0.1 - 1.8, 0.1 - 1.7, 0.1 - 1.6, 0.1 - 1.5, 0.1 - 1.4, 0.1 - 1.3, 0.1 - 1.2, 0.1 - 1.1, 0.1 - 1.0, 0.1 - 0.9, 0.1 - 0.8, 0.1 - 0.7, 0.1 - 0.6, 0.1 - 0.5, 0.5 - 2.0, 0.6 - 2.0, 0.7 - 2.0, 0.8 - 2.0, 0.9 - 2.0, 1.0 - 2.0, 1.1 - 2.0, or 1.2 - 2.0ml, more preferably between 0.2 and 0.7 ml of the aqueous liquid per gram ceramic bone substitute powder, and in the aqueous liquid may further comprise an X- ray contrast agent (e.g. iohexol).

Also disclosed is a method for fixation of an implant comprising (or consisting essentially of):
a) mixing a dry powder consisting essentially of a calcium phosphate component (e.g. hydroxyapatite) and/or a calcium sulfate, and a cyclic glycopeptide, with an aqueous liquid component to form an injectable or moldable composition, wherein the amount of cyclic glycopeptide in the injectable or moldable composition ranges from 1 - 600mg/ml composition;
b) inserting the resulting composition from step a) into a bone cavity;
c) introducing an implant into the bone cavity either before or after inserting the composition in step b) ; and
d) allowing the composition to harden in vivo.

The dry powder may consist essentially of a calcium phosphate component (e.g. hydroxyapatite) in an amount ranging from 30 - 50 w/w % and calcium sulfate in an amount ranging from 50-70 w/w%.

The method may further include, prior to step (a), the step of preparing a bone cavity suitable for receiving a prosthesis, or the step of removing a prosthesis from the mammal in need of revision arthroplasty to provide the bone cavity. The method is suitably performed on a mammal, such as a human, dog, horse or cat suffering from a muscular skeletal disorder.

The powder component, cyclic glycopeptide component and aqueous liquid component are provided in sterile form, suitable for use in the therapeutic method of the invention that requires aseptic conditions. The cyclic glycopeptide component may be a cyclic glycopeptide antibiotic for example one or more selected from vancomycin, eremomycin, ristocetin A, teicoplanin, telavancin, bleomycin, ramoplanin, and decaplanin; more preferably selected from among vancomycin, eremomycin, ristocetin A, teicoplanin, and telavancin.

The composition, comprising a mixture of a ceramic bone substitute powder component and an aqueous liquid and a cyclic glycopeptide component is prepared by a mixing step. The mixing, under aseptic conditions, may be carried out manually in a sterile mixing tool. According to one embodiment, the composition in the sterile mixing tool may then be introduced into one or more injection syringe which may be used for introducing the composition into the bone cavity (WO2005/122971). An initial mixing time of 15 seconds to one minute, for example about 30 seconds has been found suitable when carrying out the present invention. Following mixing of the powder component, cyclic glycopeptide component and the aqueous liquid, a composition is formed which begins to harden. In the present context the expressions "harden" and "hardened" are used to designate a setting reaction taking place when hydraulic cements, such as bone substitute powder, react with water. When the ceramic bone substitute composition comprises HA, it is preferred to use HA that has been subjected to an additional heating step, as described above (PCT/EP2014/053330) to ensure hardening of the mixed composition comprising a cyclic glycopeptide. Alternatively, the powder components of the ceramic bone substitute composition comprising HA are mixed with the liquid component to form a paste in a first step, and then the cyclic glycopeptide component is added and mixed with the paste in a second step as described in WO2011098438A1 to ensure hardening of the paste.

The ceramic bone substitute composition allows a sufficient working time for delivering the composition, prior to its hardening. The ceramic bone substitute composition may be introduced into the bone cavity by any suitable delivering tool, for example by using an injection syringe. A syringe for delivering the composition is provided with a cannula having a suitable cannula gauge, for example 16G. When using a 16G cannula syringe, the working time for delivering the ceramic bone substitute composition is approximately 6 minutes. This time window leaves a sufficiently broad time span for mixing and injecting the bone substitute composition, and allows for minor delays often occurring during surgery. Where the ceramic bone substitute composition is introduced into the bone cavity subsequent to insertion of the implant, the use of an injection syringe loaded with the composition may be particularly suitable.

The inserted implant (e.g. screw, pin, nail, wire, plate, rod, and primary or revision joint prosthesis) is introduced into the bone cavity, where it comes in contact with the ceramic bone substitute composition injected or molded into the cavity. Since the final setting time of the ceramic bone substitute composition from first mixing is about 8 to 20 minutes, this leaves a sufficient time window for introducing both the ceramic bone substitute composition and the implant into the bone cavity before the composition hardens.

The composition is for use in the treatment of mammals with bone fractures, re-setting bones, as well as mammals in need of primary or revision arthroplasty. Load-bearing primary or revision prostheses may be used for the hip, knee, shoulder, finger, ankle, wrist and elbow joint. Revision arthroplasty involves the replacement of a failed prosthesis, where the failed prosthesis may be a primary prosthesis or may be a revision prosthesis. The therapeutic method may include the additional step of creating a bone cavity for receiving the bone substitute composition and the prosthesis, or may include the additional step of removing a failed pre-existing prosthesis. The therapeutic method employing the composition of the invention is useful for the treatment of a human, dog, cat or horse having a musculoskeletal disorder; and in particular a human.

Preparation of the bone cavity may require removal of damaged bone and in the case of removing a pre-existing prosthesis this may include removal of PMMA cement in order to provide a bone cavity suitable for receiving and fixation of the prosthesis. Suitably, a straight box or offset chisel may be used to determine the orientation of the canal of the bone into which a prosthesis is to be implanted, and to clear the canal for acceptance of a starter reamer. A single starter reamer on a T-handle may then be used to initiate an opening into the distal portion of the bone canal, where the reamer is introduced to a level appropriate to the size prosthesis templated on the pre-operative X-rays. One or more broach of increasing size may then be used to enlarge the proximal portion of the bone canal, until the template implant size is reached, which is selected to ensure a tight fit for the prosthesis to be implanted. After the bone substitute composition has been introduced into the cavity, the prosthesis stem can be tapped into position with a press fit.

The primary or revision prosthesis introduced into the bone cavity typically has a stem or protruding peg, which may be fluted and/or tapered, and whose dimensions are selected to achieve a close fit when impacted into the cavity. The selected prosthesis is itself normally an uncoated prosthesis, and typically has a stem made from metal, for example titanium alloy, or cobalt-chromium-molybdenum (CoCrMo) alloy. The prosthesis may have a polished surface or alternatively at least a part of the surface may be coated, for example a porous titanium surface coating may be applied as a porous plasma spray. If the prosthesis is at least partially coated, additional suitable coating materials include a calcium phosphate coating such as a hydroxyapatite coating. Typically the distal stem region of the prosthesis has a polished surface. A hip prosthesis may be a modular prosthesis or monoblock prostheses and may have a long stem, or a standard length stem for insertion into a femoral bone cavity. A knee prosthesis comprises a tibial implant which has a modular stem and optionally pegs that are inserted into corresponding cavities in the resected tibial surface, which serve to secure a modular tibial tray to the tibial bone. A full knee prosthesis further comprises a patella portion mounted on the femur, which may be secured to the femur by means of a peg, extending from the patella portion, that is inserted into a femoral cavity. A shoulder prosthesis comprises a humeral implant which has a modular stem that is inserted into a corresponding cavity in the resected humeral surface and serves to secure a modular humeral tray to the humerus. Corresponding glenoid components are mounted on the glenoid bone, that can be secured by means of a peg, extending from the glenoid component, that is inserted into a glenoid cavity.

Prostheses used in bone fracture surgery, such as a screw, pin, nail, wire, plate or rod, are typically manufactured from steel, titanium alloy or cobalt alloy.

The treatment of mammals with bone fractures, or mammals in need of primary or revision arthroplasty of joints, needs to address the problems caused by micromotion, which recent studies have shown to be a major cause of prosthesis failure (Wazen RM1 et al (2013)). Micromotion at the interface between the implant and bone is a function of the amount of implant in contact with host bone, the strength of the bone in direct contact with the implant and the coefficient of friction between the two surfaces. Above a low cut-off level, repetitive micromotion inhibits bone growth and leads to later loosening of an implant.

The effective fixation of an implant in the form of a screw, pin, wire, plate or nail in bone material, in particular cancellous bone material requires a tight fit within the bone cavity, but in addition relies on direct contact between the entire surface of the inserted implant and the walls of the bone cavity. A cavity introduced in the cell-like structure of cancellous bone will not always have walls that are sufficiently uniform to allow direct contact between bone material and the implant to be achieved. Poor fixation of an implant during surgery may lead to immediate implant loosening or dislocation, while poor fixation will be the cause of micromotion that will compromise bone healing and eventually lead to implant loosening and displacement. The injectable composition of the invention has fluid properties enabling it to fill out the space between the surface of an implant and the walls of the bone cavity into which the implant is inserted. The composition hardens within about 20 minutes from mixing such that each implant inserted by a surgeon is firmly fixed within the 'real-time' of a surgical procedure, and can resist the pull-out forces (comprising tensional and/or shear forces) that take place when aligning fractured bones. The increased fixation strength of the one or more inserted implants also minimizes the risk of micro-motion inhibiting effective bone re-growth during the days following surgery.

The mechanical fixation of the stem of a primary or revision prosthesis, in most cases, relies on a short region located towards the tip of the tapered stem whose dimensions secure a precise tight fit with the bone cavity into which it is inserted. As much as 80 to 90% of the surface of the prosthesis immediately following implantation will often not be in direct contact with the walls of the bone cavity, being separated by a gap of up to 1 or more mm. The risk of micromotion would be reduced if a larger proportion of the prosthesis stem could make tight contact to the walls of the bone cavity. Here also the injectable or moldable composition of the invention, will fill out a gap of up to 1 or more mm between the stem of the prosthesis and the bone cavity prior to hardening, and following hardening will provide enhanced fixation over the full length of the tapered stem in the bone cavity.

The fixation of revision prostheses is similarly enhanced by introducing the composition into the bone cavity, where direct contact between the bone cavity of the revision prosthesis is limited to a short region towards the end of the prosthesis stem. Revision arthoplasty requires the removal of a primary or revision prosthesis and any remaining PMMA-type cement, and preparation of an extended bone cavity to receive the revision prosthesis, which often leads to cracks and irregularities in the upper part of the bone cavity. The composition of the invention, by virtue of its ability to fill these cracks and irregularities prior to hardening, ensures an immediate fixation of revision prosthesis with enhanced pull-out strength.

### Examples

In the present study, we show that an injectable and hardenable calcium sulfate/hydroxyapatite bone substitute composition containing a cyclic glycopeptide will enhance the fixation of an implant in an artificial-cancellous bone tissue, providing greater pull-out strength, as compared to the bone substitute composition alone. Materials used in the study are listed below:

### Powders

The purities of the synthetic Calcium sulfate hemihydrate (CSH) and Calcium sulfate dihydrate (CSD) used in the examples met the test requirements stated both in the monograph "Calcium Sulfate Dihydrate" 01/2002:0982, European Pharmacopoeia and in the "Official Monograph for Calcium Sulfate" U.S. Pharmacopoeia 25/National Formulary 20. The particle size distribution of the CSH was from 0.1 - 80 µm. The particle size distribution of the accelerator CSD was from 0.1 to 100 µm.

The hydroxyapatite (HA) powder used in the examples has been produced by a precipitation reaction, sintered at a high temperature (1275 ± 50°C for 4 h) and micronized. The HA in the powder component used in combination with the cyclic glycopeptide component had additionally been further heat treated at 500°C for 2 h (PCT/EP2014/053330). The HA powder met the specification ASTM F1185-03 "Standard Specification for Composition of Hydroxylapatite for Surgical Implants" and ISO 13779-1 "Implants for surgery -- Hydroxyapatite -- Part 1: Ceramic hydroxyapatite". The particle size distribution of the HA was from 0.1 to 35 µm with a specific surface area < 10 m²/g.

### Liquid phase

In the Examples, either iohexol solution or saline have been used as the liquid phase.
The iohexol solution used consisted of water for injection (WFI), Iohexol, the buffer Trometamol (Tris: tris(hydroxymethyl)aminomethane) and the chelating agent Edetate Calcium Disodium (calcium EDTA). The iohexol solution met the requirements stated in the US Pharmacopoeia for Iohexol Injection. In addition, the content of iohexol, trometamol and sodium calcium edetate met each specific requirement according to standards.

The saline solution consisted of 0.9 wt % NaCl in water for injection (WFI). The saline used met the requirements stated in the Ph EP 0193 Sodium Chloride.
The reason for having a solution comprising iohexol or similar X-ray agent as the liquid phase was to increase the radiopacity of the bone substitute material (see WO 03/053488).

### Additional organic compounds tested for enhancement of implant fixation:

In the examples the addition of two compounds have been tested, gentamicin sulfate and vancomycin hydrochloride. Gentamicin sulfate [CAS 1405-41-0] is known as a broad-spectrum aminoglycoside antibiotic derived from an Actinomycete that can be used in the treatment of various infections caused by organisms sensitive to gentamicin, especially gram-negative organisms. The gentamicin sulfate met the requirements stated in the Ph EP Gentamicin Sulfate RS.

Vancomycin hydrochloride [CAS 1404-93-9] is known as a cyclic glycopeptide antibiotic for use against gram-positive bacteria, including *Staphylococcus aureus, Staphylococcus epidermidis,* alpha and beta haemolytic streptococci, group D streptococci, corynebacteria and clostridia. The vancomycin hydrochloride met the requirements stated in the Ph EP Vancomycin hydrochloride.

### General properties

By combining HA and CSH, an optimal balance is achieved between synthetic bone substitute resorption rate and bone in-growth rate. CSH is converted to CSD during the setting process. CSD acts as a resorbable carrier for HA. HA has a slow resorption rate, high osteoconductivity promoting bone in-growth and gives long term structural support to the newly formed bone.

### Example 1

### Preparation of injectable biphasic ceramic bone substitute composition

In this Example, 3 different types of hardenable ceramic bone substitute materials have been prepared. All three samples consisted of 59.6 wt% α-CSH, 40.0 wt% HA, 0.4 wt% CSD and the same liquid-to-powder ratio (L/P=0.43 mL/g), but the liquid phase as well as the type of compound added was varied, see Table below.

| **Sample name** | **Liquid phase** | **Added compound** |
|---|---|---|
| CSH/HA | Iohexol (180 mg I/mL) | - |
| CSH/HA + Genta | Saline | Gentamicin sulfate |
| CSH/HA + Vanco | Iohexol (180 mg I/mL) | Vancomycin hydrochloride |

### CSH/HA

11.6 g of the ceramic bone substitute was mixed with 5.0 mL of a liquid phase containing iohexol (180 mg I/mL), i.e. giving a L/P ratio of 0.43 mL/g. The mixing was conducted for 30 seconds using a specially designed mixing and injection device (WO 2005/122971). The obtained paste could be injected with a 16 G needle for up to 5 min and be molded by hand between 5 and 7 minutes. The initial setting time of the paste was 8 min and the final setting time 15 min (evaluated with Gillmore needles; ASTM C266). The maximum setting temperature was 38°C (ASTM F451). The wet compressive strength of bars with 8 mm height and 4 mm diameter (after 24 h in Ringer solution) was 6-11 MPa.

### CSH/HA + Gentamycin

9.3 g of the ceramic bone substitute was mixed with 4.0 mL of a liquid phase containing saline and 200 mg pre-dissolved Gentamicin sulfate (corresponding to 30 mg Gentamicin/mL solution); giving a concentration of 17.5 mg Gentamicin/mL paste. The L/P-ratio was 0.43 mL/g.

The mixing was conducted for 30 seconds using a specially designed mixing and injection device (WO 2005/122971). The obtained paste could be injected with a 16 G needle for up to 6 min. The initial setting time of the paste was 8 min and the final setting time 10 min (evaluated with Gillmore needles; ASTM C266). The maximum setting temperature was 37°C (ASTM F451). The wet compressive strength of bars with 8 mm height and 4 mm diameter (after 24 h in Ringer solution) was 9-12 MPa.

### CSH/HA + Vancomycin

9.3 g of the ceramic bone substitute was mixed with 4.0 mL of a liquid phase containing iohexol solution (180 mg I/mL) and pre-dissolved vancomycin hydrochloride (corresponding to 125 mg vancomycin/mL solution); giving a concentration of 66 mg vancomycin/mL paste. The L/P-ratio was 0.43 mL/g.

The mixing was conducted for 30 seconds using a specially designed mixing and injection device (WO 2005/122971).The obtained paste could be injected with a 16 G needle for up to 7 min and be molded by hand between 6 and 8 minutes. The initial setting time of the paste was 7 min and the final setting time 12 min (evaluated with Gillmore needles; ASTM C266). The maximum setting temperature was 39°C (ASTM F451). The wet compressive strength of bars with 8 mm height and 4 mm diameter (after 24 h in Ringer solution) was 4-7 MPa.

All three types of CSH/HA bone substitutes had similar performance in that the initial wet compressive strength of all samples was in the same range as the compressive strength of cancellous bone (1-20 MPa).

### Example 2

### Use of a model system to demonstrate that an injectable biphasic ceramic bone substitute composition comprising vancomycin enhances the fixation of an implant

The effect of injectable compositions, prepared according to example 1, on the fixation of an implant was determined in a model system by determining the pull-out strength and resistance to torsional forces of screws inserted into a cancellous bone model that has been augmented with the injectable composition.

The cancellous bone model comprised a rigid open cell foam block (product no. 1522-507) supplied by Sawbones® (Sawbones.com). The foam block has a cell structure that is over 95% open, with a cell size is 1.5 to 2.5 mm resembling that of human cancellous bone, making it suitable for dynamic testing or cement injection. The foam block has a density of 0.12g/cc, a compressive strength is 0.28 MPa and compressive Modulus is 18.6 MPa, which is relatively low in order, and was used because it most closely mimicks osteoporotic bone where fixation of implants is particularly difficult.

### Experimental set-up

The bone model comprised a 81cm³ block (Figure 1A) cut from the foam block (Sawbones®); and a 2mm thick plexiglas plate, simulating the compact cortical layer of bone. The plate was perforated by a single hole drilled centrally through the plate, allowing for the screw to pass through the plate but not attach to it (Figure 1B). A 2 cm deep hole was also pre-drilled in the block using a 3.5 mm drill bit. A threaded guidewire (having dimensions of 2.0x150mm) was used to pre-locate the correct position in the foam block for the subsequent precise placement of a cannulated screw. The screw, a partially threaded 5.0x60mm long screw made of steel, and the guidewire (Asnis™ III) were supplied by Stryker (Footandanklefixation.com). The location of the inserted screw in the foam block in the experimental set up is shown in Figure 1C.

### Experimental procedure

The biphasic ceramic bone substitute compositions ("CSH/HA", "CSH/HA + Genta" or "CSH/Vanco") were prepared and mixed as described in Example 1. A volume of the mixed composition (∼4mL) was injected into the pre-drilled hole in the foam block, mounted beneath the plexiglas plate. The composition was injected into the block, using a 16G cannula syringe, at 3 minutes after start of mixing the components of the composition. The guidewire was then inserted into the foam block (following the same channel as the injected composition) within 4 minutes after start of mixing the composition components, followed by placement of the screw (Figure 2). The composition, with the screw inserted, was allowed to harden, corresponding to 20 minutes from mixing the composition components.

The foam blocks into which the screws were inserted, were maintained at 37°C under wet conditions, by application of 300mL deionized water per block in order to mimick in-vivo conditions.

A total of 10 inserted screws (inserted into foam block) for each of the 4 tested fixation conditions (+/- augmentation with 3 tested bone substitute compositions) were tested for tensional pull-out strength.

The pullout force was measured with an "MTS Insight 5 single column material testing workstation" equipped with a 500N load cell, supplied by MTS Systems Corporation, 14000 Technology Drive, Eden Prairie, MN USA 55344. The equipment is designed for single axis-tension testing. The axial fixation strength of the inserted screws, as illustrated in Figure 3, was tested by subjecting them to tensional stress at a pre-set pull-out speed of 5mm/min (Figure 4).

The fixation of the inserted screws was further evaluated, as illustrated in Figure 7, by inducing shear forces and torsion forces. Torsion forces can be measured by means of a torque driver by unscrewing the implant from the augmented foam. Shear stresses can also be examined in the pullout experiments by placement of the implant at different angles (e.g. 10-60°) to the direction of the induced pullout force.

### Experimental results

### I. Pullout profiles for screws inserted in the model foam block

The pull-out force required to extract a screw inserted in the model foam block is measured in Newtons (N), and is registered as a function of distance (millimeters). The profile of the tensile force needed to remove the screw shows an initial increase in force as the screw is contained within the model foam block, followed by a drop in force once the screw detaches from the sample (Figure 5A).

### II. Screws inserted with augmentation with a bone substitute composition

Augmentation of the insertion of the screw with a bone substitute composition in all cases increased the tensile force required to raise the inserted screw from the model foam block, when compared to "control samples" where the screws were inserted without a bone substitute composition (Figure 5B versus Figure 5A; and Figure 6). The addition of gentamicin to the ceramic bone substitute composition ("CSH/HA + Genta") further slightly increased the tensile force required to raise the inserted screw from the model foam block (Figure 5C versus Figure 5A).

The use of a vancomycin supplemented ceramic bone substitute composition, by comparison, gave a significantly greater increase in the pull-out force under wet conditions as compared to any of the other tested compositions (Figure 5D; and Figure 6). In addition the tensile profile in Figure 5D reveals that the force required to raise the inserted screw remains high over a greater distance. Thus when the inserted screw is augmented with the vancomycin supplemented ceramic bone substitute composition, the distance the screw can be raised before losing its tensile strength is greater than in the case of the ceramic bone substitute composition alone.

The mean values (±SD) for all experimental setups are summarized in Figure 6 (n=10).
III. Resistance to shear and torsion forces of screws inserted in the model foam block The use of a vancomycin supplemented ceramic bone substitute composition was also observed to produce significantly greater resistance to the shear and torsion forces required to remove the inserted screw from the model foam block maintained under wet conditions, as compared to any of the other tested compositions.

Regarding the examination of shear forces, screws are inserted in the model foam block at angles of 10-60° relevant to the direction of the induced pull-out force. The force required to remove the implant from the augmented foam consists in this case of a combination of tensile and shear forces that are transferred to the augmented area.
Furthermore, the resistance to torsion forces is evaluated by unscrewing the implant from the augmented foam block using a torque driver.

### Example 3 Setting performance of an injectable biphasic ceramic bone substitute composition comprising vancomycin

The following tests demonstrate the effect of the vancomcyin content of an injectable ceramic bone substitute on its setting properties, within a concentration range of 33-132 mg vancomycin/mL paste. In these tests, the ceramic bone substitute consisted of 59.6 % CSH, 40 % HA and 0.4 % CSD and the L/P ratio was 0.43 mL/g. Three different types of liquid phases were investigated. The setting time was analyzes with Gillmore needles, ASTM C266. The results are found in Table 1.

**Table 1: Setting properties of ceramic bone substitute comprising different amounts of Vancomycin.**

| **Type of liquid phase** | **Amount of Vancomycin (mg/mL)** | **Initial setting time, IST (min)** | **Final setting time, FST (min)** | **Moldability start** | **Injectable (min)** |
|---|---|---|---|---|---|
| Iohexol solution, 180 mg I/mL (CERAMENT™IC-TRU) | 33 | 6.5 | 10.5 | 5 min | 5 |
| | 66 | 7 | 12 | 4 min 45 s | 5 |
| | 132 | 6 | 9 | 4 min | 3 |
| Sterile water (WFI) | 33 | 7.5 | 11 | 7min | 7 |
| | 66 | 8.5 | 13 | 5 min 15s | 7.5 |
| | 132 | 7 | 9.5 | 4 min 50s | 5 |
| Saline (9 mg NaCl/mL) | 33 | 12 | 14.5 | 10 min | 8 |
| | 66 | 10.3 | 13.8 | 7min 45s | 8 |
| | 132 | 6.5 | 10 | 4min 30s | 4 |

The results from these tests show that concentrations of vancomycin in the range of 33-132 mg/mL paste all gave acceptable setting performance for an injectable bone substitute.

### References cited

Liskamp et al 2008 Modern Supramolecular Chemistry: Strategies for Macrocycle Synthesis. Edited by François Diederich, Peter J. Stang, and Rik R. Tykwinski WILEY-VCH Verlag GmbH & Co. KGaA, Weinheim
Procter P., Hess B., Murphy M., Phelps R.C., Miles A.W., Gheduzzi S. (2008) In-vitro study of screw fixation in augmented cancellous bone models. 54th Annual Meeting of the Orthopaedic Research Society, Poster Nr. 1720.
Pijls, BJ., Valstar, ER., Nouta, K-A., Plevier, JWM., Fiocca, M., Middeldorp, S., and RGHH Nelissen (2012) Early migration of tibial components is associated with later revision. Acta Orthopaedica 83(6):614-624.
Weiss, RJ., Stark, A., Kärrholm J., (2011) A modular cementless stem vs. cemented long-stem prostheses in revision surgery of the hip. Acta Orthopaedica 82(2):136-142.
Wazen RM1, Currey JA, Guo H, Brunski JB, Helms JA, Nanci A.(2013) Micromotion-induced strain fields influence early stages of repair at bone-implant interfaces. Acta Biomater. 9(5):6663-74.
Zampelis V., M. Tägil, L. Lidgren, H. Isaksson, I. Atroshi, and J-S Wang (2013) The effect of a biphasic injectable bone substitute on the interface strength in a rabbit knee prosthesis model J Orthop Surg Res. 8: 25.

## Claims

1. Use of a cyclic glycopeptide to enhance the resistance of a composition following *in vivo* hardening to one or more of a tensile, shear and torsional force,
wherein the composition comprises a powder component and an aqueous liquid component and a cyclic glycopeptide component; wherein the powder component comprises a calcium sulfate component and/or a calcium phosphate component; and wherein the powder component and the aqueous liquid component and the cyclic glycopeptide component on mixing forms an injectable or moldable composition capable of hardening *in vivo.*

2. The use of the cyclic glycopeptide according to claim 1, wherein the cyclic glycopeptide component is a dry component or is a component of the aqueous liquid component.

3. The use of the cyclic glycopeptide according to claims 1 or 2, wherein the cyclic glycopeptide component is selected from vancomycin, eremomycin, ristocetin A, bleomycin, ramoplanin, telavancin, decaplanin and teicoplanin.

4. The use of the cyclic glycopeptide according to any of claims 1 to 3, wherein the calcium sulfate component comprises α-calcium sulfate hemihydrate and additionally an accelerator, wherein the accelerator is selected from among calcium sulfate dihydrate and sodium chloride.

5. The use of the cyclic glycopeptide according to any of claims 1 to 4, wherein the calcium phosphate component is hydroxyapatite or the calcium phosphate component is tricalcium phosphate and a phosphate salt or a hardened calcium phosphate.

6. The use of the cyclic glycopeptide according to any of claims 1 to 5, wherein the powder component comprises a calcium sulfate component and a calcium phosphate component.

7. The use of the cyclic glycopeptide according to claim 6, wherein the powder component consists of 50 to 70 wt/wt% calcium sulfate component and 30 to 50 wt/wt% hydroxyapatite, and the liquid component comprises 2-1250 mg vancomycin /ml solution.

8. The use of the cyclic glycopeptide according to claim 7, wherein the composition on mixing comprises between 0.1 and 2 ml, preferably between 0.2 and 0.7 ml of the aqueous liquid per gram powder.

9. The use of the cyclic glycopeptide according to any of claims 1 to 8, wherein the aqueous liquid comprises an X- ray contrast agent.

10. The use of the cyclic glycopeptide according to claims 8 or 9, wherein the composition on mixing comprises 1-600mg vancomycin/ml injectable and/or moldable composition.

## Patentansprüche

1. Verwendung eines cyclischen Glykopeptids zur Erhöhung der Beständigkeit einer Zusammensetzung nach In-Vivo-Härtung gegenüber einer oder mehreren von einer Zug-, Scher- und Torsionskraft,
wobei die Zusammensetzung eine Pulverkomponente und eine wässrige flüssige Komponente und eine cyclische Glykopeptidkomponente umfasst; wobei die Pulverkomponente eine Calciumsulfatkomponente und/oder eine Calciumphosphatkomponente umfasst; und
wobei die Pulverkomponente und die wässrige flüssige Komponente und die cyclische Glykopeptidkomponente beim Mischen eine injizierbare oder formbare Zusammensetzung bildet, die In-Vivo härten kann.

2. Verwendung des cyclischen Glykopeptids nach Anspruch 1, wobei die cyclische Glykopeptidkomponente eine trockene Komponente ist oder eine Komponente der wässrigen flüssigen Komponente ist.

3. Verwendung des cyclischen Glykopeptids nach den Ansprüchen 1 oder 2, wobei die cyclische Glykopeptidkomponente ausgewählt ist aus Vancomycin, Eremomycin, Ristocetin A, Bleomycin, Ramoplanin, Telavancin, Decaplanin und Teicoplanin.

4. Verwendung des cyclischen Glykopeptids nach einem der Ansprüche 1 bis 3, wobei die Calciumsulfatkomponente α-Calciumsulfat-Halbhydrat und zusätzlich einen Beschleuniger umfasst, wobei der Beschleuniger ausgewählt ist aus Calciumsulfatdihydrat und Natriumchlorid.

5. Verwendung des cyclischen Glykopeptids nach einem der Ansprüche 1 bis 4, wobei die Calciumphosphatkomponente Hydroxyapatit ist oder die Calciumphosphatkomponente Tricalciumphosphat und ein Phosphatsalz oder ein gehärtetes Calciumphosphat ist.

6. Verwendung des cyclischen Glykopeptids nach einem der Ansprüche 1 bis 5, wobei die Pulverkomponente eine Calciumsulfatkomponente und eine Calciumphosphatkomponente umfasst.

7. Verwendung des cyclischen Glykopeptids nach Anspruch 6, wobei die Pulverkomponente aus 50 bis 70 Gew./Gew.-% Calciumsulfatkomponente und 30 bis 50 Gew./Gew.-% Hydroxyapatit besteht und die flüssige Komponente 2 - 1250 mg Vancomycin /ml Lösung umfasst.

8. Verwendung des cyclischen Glykopeptids nach Anspruch 7, wobei die Zusammensetzung beim Mischen zwischen 0,1 und 2 ml, vorzugsweise zwischen 0,2 und 0,7 ml der wässrigen Lösung pro Gramm Pulver umfasst.

9. Verwendung des cyclischen Glykopeptids nach einem der Ansprüche 1 bis 8, wobei die wässrige Flüssigkeit ein Röntgenkontrastmittel umfasst.

10. Verwendung des cyclischen Glykopeptids nach den Ansprüchen 8 oder 9, wobei die Zusammensetzung beim Mischen 1 - 600 mg Vancomycin /ml injizierbarer und/oder formbarer Zusammensetzung umfasst.

## Revendications

1. Utilisation d'un glycopeptide cyclique pour augmenter la résistance d'une composition suite au durcissement *in vivo* à une ou plusieurs parmi des forces de traction, de cisaillement et de torsion, dans laquelle la composition comprend un constituant pulvérulent et un constituant liquide aqueux et un constituant glycopeptide cyclique; dans laquelle le constituant pulvérulent comprend un constituant sulfate de calcium et/ou un constituant phosphate de calcium ; et dans laquelle le constituant pulvérulent et le constituant liquide aqueux et le constituant glycopeptide cyclique, lors du mélange, forment une composition injectable ou moulable apte à durcir *in vivo.*

2. Utilisation du glycopeptide cyclique selon la revendication 1, dans laquelle le constituant glycopeptide cyclique est un constituant sec ou est un constituant du constituant liquide aqueux.

3. Utilisation du glycopeptide cyclique selon les revendications 1 ou 2, dans laquelle le constituant glycopeptide cyclique est sélectionné parmi la vancomycine, l'érémomycine, la ristocétine A, la bléomycine, la ramoplanine, la télavancine, la décaplanine et la téicoplanine.

4. Utilisation du glycopeptide cyclique selon l'une quelconque des revendications 1 à 3, dans laquelle le constituant sulfate de calcium comprend un semi-hydrate de sulfate de calcium α et additionnellement un accélérateur, dans laquelle l'accélérateur est sélectionné parmi un dihydrate de sulfate de calcium et un chlorure de sodium.

5. Utilisation du glycopeptide cyclique selon l'une quelconque des revendications 1 à 4, dans laquelle le constituant phosphate de calcium est l'hydroxyapatite ou le constituant phosphate de calcium est le phosphate de tricalcium et un sel de phosphate ou un phosphate de calcium durci.

6. Utilisation du glycopeptide cyclique selon l'une quelconque des revendications 1 à 5, dans laquelle le constituant pulvérulent comprend un constituant sulfate de calcium et un constituant phosphate de calcium.

7. Utilisation du glycopeptide cyclique selon la revendication 6, dans laquelle le constituant pulvérulent est constitué à 50 à 70% en poids du constituant sulfate de calcium et à 30 à 50 % en poids d'hydroxyapatite, et le constituant liquide comprend de 2 à 1 250 mg de vancomycine/ml de solution.

8. Utilisation du glycopeptide cyclique selon la revendication 7, dans laquelle la composition, lors du mélange, comprend entre 0,1 et 2 ml, de préférence entre 0,2 et 0,7 ml du liquide aqueux par gramme de poudre.

9. Utilisation du glycopeptide cyclique selon l'une quelconque des revendications 1 à 8, dans laquelle le liquide aqueux comprend un agent de contraste de radiographie.

10. Utilisation du glycopeptide cyclique selon les revendications 8 ou 9, dans laquelle la composition, lors du mélange, comprend de 1 à 600 mg de vancomycine/ml de composition injectable et/ou moulable.
